Europäisches Patentamt

European Patent Office

Office européen des brevets

(11) Publication number: 0 277 773
A1

## EUROPEAN PATENT APPLICATION

(21) Application number: 88300746.0

(22) Date of filing: 28.01.88

(51) Int. Cl.⁴: C 12 N 15/00
A 61 K 39/245

(30) Priority: 30.01.87 US 9273

(43) Date of publication of application:
10.08.88 Bulletin 88/32

(84) Designated Contracting States:
AT BE CH DE ES FR GB GR IT LI LU NL SE

(71) Applicant: THE BOARD OF TRUSTEES OF THE LELAND
STANFORD JUNIOR UNIVERSITY
Encina 105 Stanford University
Stanford, California 94305 (US)

(72) Inventor: Mocarski, Edward S.
141 Erica Way
Menlo Park California 94025 (US)

Spaete, Richard R.
2501 Coronet Boulevard
Belmont California 94002 (US)

(74) Representative: Harrison, David Christopher et al
MEWBURN ELLIS & CO 2/3 Cursitor Street
London EC4A 1BQ (GB)

Claims for the following Contracting States: ES + GR.

(54) Hybrid cytomegalovirus (CMV) and vaccine.

(57) Attenuated chimeric cytomegaloviruses are provided,
where the virus carries genetic information for transcription or
translation of products in vitro or in vivo. Particularly, the
chimeric CMV may be used as a vaccine for providing
protection for CMV as well as the pathogens, where the
chimeric CMV carries genetic information for expression of
antigens associated with such pathogens.

EP 0 277 773 A1

# Description

## HYBRID CYTOMEGALOVIRUS (CMV) AND VACCINE

Vaccines are provided employing cytomegalovirus as the vector and a sequence encoding an epitope of interest inserted into the vector. The vaccines primarily find use in humans.

For many diseases, the cause is the failure of the immune system to overcome the invasion and proliferation of a pathogen. Since the immune system has a memory as to prior infections, the response of the immune system can be enhanced by subjecting the host to an attenuated version of the pathogen or, in some instances, one or more components of the pathogen or fragments of such components. These vaccines, by activating the immune system and stimulating memory, allowing the host to more rapidly respond to an infection and limit the infection, thus preventing disease.

For the most part, inoculating a host with a protein component of a pathogen does not induce a strong, lasting immune response. This appears to result from the short half-life of the protein, the small amount which one can inject and the unnatural presentation of a protein to the host as compared with the manner in which the protein is presented in the pathogen. Therefore, it has been a goal to find ways to enhance the immune response by using an attenuated version of the pathogen as a vaccine. However, other means have become available whereby the antigen may be presented in an environment which is similar to the environment of the natural antigen in the pathogen. There is, therefore, substantial interest in finding safe ways to prepare vaccines where antigens may be presented which will provide strong, lasting immune responses to prevent infectious diseases.

## DESCRIPTION OF THE RELEVANT LITERATURE

Spaete and Mocarski, J. Virol. (1985) 56:135; Geballe, Ibid (1986) 57:864; and Geballe et al., Cell (1986) 46:865 describes the structure and nature of expression of cytomegalovirus. Wathen and Stinski, J. Virol. (1982) 41:462; McDonough and Spector, Virology (1983) 125:31; McDonough et al., J. Virol., (1985) 53:711; and Hutchinson et al., Virology (1986) 155:160 describe the terminal repeat areas of the cytomegalovirus (CMV). Pennock et al. Mol. Cell. Biol. (1984) 4:399; Chakrabarti et al., Ibid (1985) 5:3403 report the insertion of the lacZ gene into baculovirus and vaccinia virus. The use of CMV (Towne) has been tested as a vaccine in human trials (Glazer et al., Ann. Intern. Med. (1978) 91:676; Quinan et al., Ibid (1984) 101:478; and Plotkin et al., Lancet i (1984) 528). This virus does not appear capable of latent infection (Plotkin & Huang, J. Infect.Dis. (1985) 152:395. See also U.S. Patent Nos. 3,959,466 and 4,058,598.

## SUMMARY OF THE INVENTION

Novel cytomegalovirus vectors, chimeric constructs, and vaccines are provided, where foreign open reading frames are inserted into the major β-gene of CMV under the control of appropriate regulatory sequences for expression. The chimeric virus may then be used for infection of a mammalian host with expression of the attenuated virus and the foreign protein to induce a safe immune response to the foreign protein. Proteins from a wide variety of pathogens may be inserted into the CMV safely to provide activation of the immune system for humoral and cellular protection.

## BRIEF DESCRIPTION OF THE FIGURE

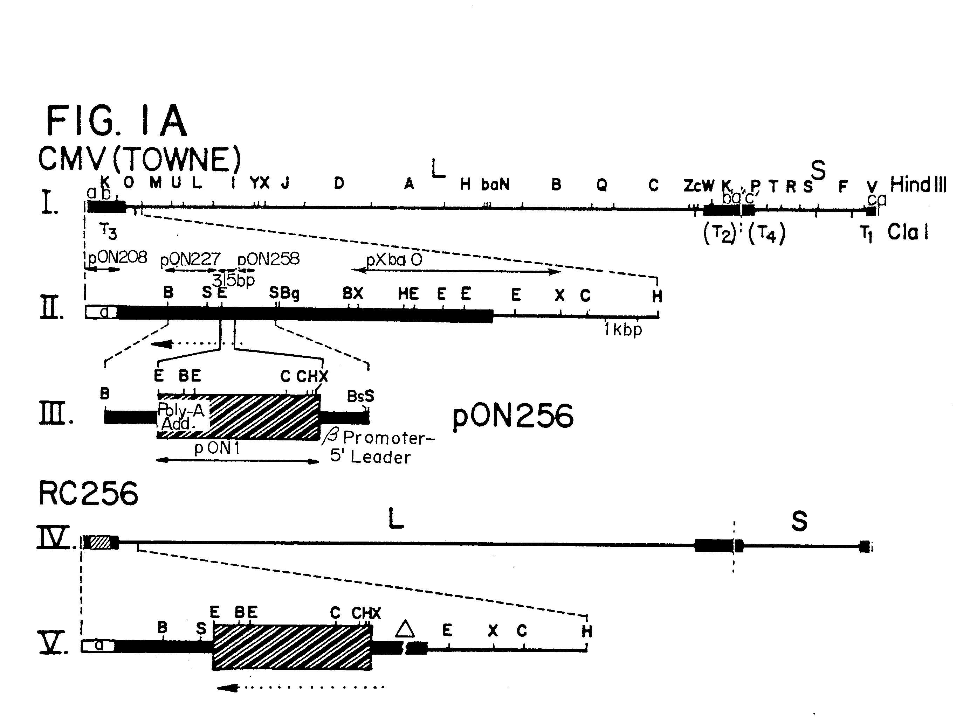

Fig. 1 shows the following:

I. Sequence arrangement of the 240 kbp CMV (Towne) genome - showing the L and S components in one (the prototypic) of four possible orientations.

II. The expanded region shows the HindIII K 9.7 kpb and 0 7.8 kbp fragments.

III. A fragment of pON256 constructed by inserting a 5.9 kbp EcoRI partial digest fragment from pON241, containing the β-promoter/regulatory and 5' leader sequences fused to lacZ protein coding and SV40 polyadenylation sequences, into the unique EcoRI site of pON227, containing 1.6 kbp of β-gene 3' flanking sequence.

IV. A diagrammatic representation of RC256 showing the lacZ/SV40 insertion as a diagonally striped block.

V. Expanded region showing a restriction map of inserted and deleted sequences within RC256 DNA.

In I, a complete HindIII map (LaFemina and Hayward, in Persistent Viruses, R. Jaenisch et al., Eds. Academic Press, New York (1980), p. 39; Ihara et al., Arch. of Virology (1986) 8:1) and ClaI terminal fragments are indicated.

## DESCRIPTION OF THE SPECIFIC EMBODIMENTS

Novel vectors are provided, which find particular application as vaccines where a chimeric cytomegalovirus is employed. The cytomegalovirus is an attenuated strain capable of proliferation in a mammalian host, capable of culture in cells or tissue in vitro, lacking virulence, and capable of being modified by insertion of a DNA sequence comprising an open reading frame which does not prevent the proliferation of the virus and is expressed by the cellular host when infected by the virus.

Attenuated CMV viruses, infectious for humans, may be employed for use as the vector, particularly Towne and AD169 (Elak and Stern, Lancet (1974) i:1; Neff et al., Proc. Soc. Exp. Biol. Med. (1979) 160:32) both being strains which allow for insertion of the open reading frame into an early gene (β gene), particularly into the b or b' (also referred to as $TR_L$ and $IR_L$) region of the viral genome, more particularly where the virus has at least two copies of this gene, into one of which the open reading frame is inserted. The insert may be inserted so as to provide for transcriptional and translational regulatory control by regulatory regions endogenous to the attenuated CMV, may be inserted with its own regulatory regions, which are functional in the viral host, which regulatory regions may be endogenous or exogenous to the CMV (by exogenous is intended a regulatory region from other than the CMV strain), or may be introduced so as to be independently expressed or expressed as a fused protein with an endogenous protein of the CMV. The endogenous protein may provide either the N-terminus or C-terminus, providing at least one amino acid, usually at least three amino acids, and up to and including the entire amino acid sequence of the CMV endogenous protein.

The insertion may include one or more genes (ORF), which genes may be from the same or different sources. The genes may be genomic, cDNA, synthetic, or combinations thereof. Genes may be fused one to another or be independent. The genes may or may not include introns, where the introns may or may not allow for variations in splicing. Usually, the open reading frame insertion will encode for at least 8 amino acids (24 nucleotides; nt) more usually at least 12 amino acids (36 nt) usually not more than about 1500 amino acids for each sequence encoding a single protein. The total number of proteins which may be encoded will usually not exceed 100, more usually not exceed about 50, generally ranging from about 1 to 10. The CMV vector is able to accommodate to about a 5,000 nt insertion.

If larger amounts of DNA are to be inserted, then deletion mutants may be employed by analogy to other herpes viruses. Up to about 20 kbp, usually up to about 15 kbp, can be deleted from the virus and a sequence of approximately equicvalent length introduced at an appropriate site.

The products which are encoded by the inserted DNA may be from any souce, prokaryotic, eukaryotic, including the viruses, bacteria, fungi, and vertibrate, including primate, more particularly human. Of particular interest will be proteins of pathogens, which for viruses may include core proteins, envelope proteins, nucleocapsid proteins, or other proteins or fragments thereof, which will elicit an immune response, e.g. neutralizing antibodies or cytotoxic immune cells; for cellular pathogens, surface membrane proteins, particularly outer member proteins, as well as other proteins which may be associated with inducing protective immunity. For mammalian cells, proteins of interest will primarily be proteins associated with neoplastic conditions or other disease states, which are susceptible to immune attack. Pathogens which may be the source of the open reading frame are described in U.S. Patent No. 4,174,384, beginning with column 13 and continuing to column 16, which disclosures are incorporated herein by reference.

In addition to the pathogens incorporated by reference, other pathogens of interest include various human T-lymphotrophic viruses, e.g. I, II, III (human immunodeficiency virus (HIV)), and IV, the parvovirus, B-19, the herpesvirus, HBLV, the agent of nonA/nonB hepatitis, or other newly discovered pathogens.

Concomitant with the open reading frames which encode peptides associated with pathogens or independent of such coding regions may be open reading frames encoding a wide variety of peptides which are associated with the health and functioning of the particular host. These genes may include lymphokines, interferons, colony stimulating factors, growth factors, growth inhibitors, or other physiological regulators.

The open reading frame insert may be introduced into the region of interest by any convenient means. One convenient means is to use recombination, by having the insert of interest flanked by regions substantially homologous with the region of interest in the CMV. The flanking segments providing homology will include at least about 50 bp, more usually at least 100 bp, and preferably about 1,000 bp, usually not more than about 5 kbp on each side of the insertion gene. In order to preferentially direct the insertion to one or the other of the $\underline{b}$ sequence of the L-component, one or both of the flanking sequences may extend beyond the $\underline{b}$ sequence to the adjacent $U_L$ sequence. The flanking sequences may have been contiguous or non-continguous in the CMV source, but will be in the region of interest and desirably in the proper orientation to provide for insertion in a particular orientation.

Conveniently, the regulatory signals may be derived from any of the genes of CMV. Desirably, the regulatory region which is selected should provide for a high efficiency of transcription, particularly one that provides an abundant messenger RNA. A gene of particular interest is one which may be characterized by being an early gene which encodes a 2.7 kb mRNA, which is present as the most abundant steady state polyadenylated transcript made during CMV growth. One or both of the regulatory regions may be obtained from the same or different CMV gene, that is, the 5'-promoter-regulator transcription initiation region and 3'-transcription termination region may be obtained from the same or different gene. If desired, one or both of the regulatory regions may be exogenous to the CMV and may be derived from any source, where the region is functional in the cellular host. Thus, the initiation region may be derived from initiation regions of genes of other viruses, such as Simian virus, adenovirus or papilloma virus, or from a mammalian cell, such as the regulatory regions associated with the actin, interleukin 2 or interferon genes.

The transcriptional initiation region may include not only the site for binding of the RNA polymerase and for processing of the 5'-terminus, but may also include regions for inducing expression of the open reading frame and enhancing the transcriptional rate, where the enhancers may be proximal or distal to the RNA polymerase binding site, or for the processing, stability, transport or translation of messenger RNA. Induction may be the result of temperature sensitivity, presence of a metabolite, or other chemical which can affect the repression or activation of the expression of the region.

The constructs for insertion will comprise the flanking regions native to the CMV, the transcriptional initiation and termination regulatory regions functional in the cellular host, and the open reading frame of interest under control of the transcriptional and translational regulatory regions, where the open reading frame and regulatory regions may be a single gene or a plurality of genes, either the same or different, usually different. For the most part, this construct may be depicted by the following formula, where no orientation is intended by the particular direction which is indicated, except where orientation is necessary for operability.

$FR_{5'} - (Ti - ORF - Tt)_n - FR_{3'}$:

FR intends a flanking region, where the subscripts intend the 5' or 3' regions of the construct and where the 5' and 3' regions of the flanking regions are different, each of the flanking regions including about 50 bp from the region of interest for insertion desirably at least one of the flanking regions including at least about 50 bp outside the particular region of interest for insertion, where the region of interest is repeated but flanked differently with the different copies present in the CMV;

Ti intends the transcriptional intiation region which includes the RNA polymerase binding site, any additional processing sites, such as the capping and splicing sites, and may include additional regions which provide for inducible regulation of transcriptional or posttranscriptional events and the enhancer region;

ORF intends the open reading frame which codes for the polypeptide of interest and includes translational initiation and termination signals;

Tt intends the transcriptional termination region, which provides termination of transcription as well as splicing, polyadenylation and processing signals;

Either Ti or Tt may also include all or a portion of the coding region of the natural gene associated with this region. The Ti region any include a coding region so that the ORF is in reading frame with such coding region resulting in a fused protein with the ORF as a carboxy-terminus. The Tt region may include all or a portion of the coding region normally associated with this region, so that the ORF is in reading frame with such coding region and provides a fused protein with the ORF as the amino-terminus. In some situations, the ORF may be inserted in reading frame between two different coding regions, which may be parts of the same or different genes, so as to provide a fused product with the polypeptide coded for by the ORF internal to the resulting protein. The flank ing region may be derived from any portion of the b sequence, particularly the β-gene, more particularly the 3' flanking sequence of the β-gene. The insertion may be in either orientation, preferably in the direction of transcription of the β-gene transcript.

The open reading frame of interest may be inserted into the region of interest by transfection, using a combination of DNA sequences, where one sequence comprises the intact CMV vector DNA and the other sequence comprises a DNA construct, preferably linear, containing at least a portion of an expression cassette bordered by the flanking regions. Cotransfection can be achieved employing calcium phosphate precipitation and glycerol shock or by electroporation. The two DNA molecules are introduced · according to conventional techniques into an appropriate host cell which allows for homologous recombination between the homologous regions flanking the insert ORF and the regions of the b sequence of the CMV vector. If desired, the ORF may also include a gene which allows for selection of infected host cells containing and expressing the insert, where the insert may or may not maintain stable extrachromosomal maintenance in the host cell. Therefore, by providing a method for selection, those host cells infected with the CMV containing the insert will be selected. Selection can be as a result of a gene which provides a detectable signal, e.g. an enzyme which produces a colored product, a surface membrane protein which may be detected by an antibody or other protein, or the like. Alternatively, after purification and passaging, the CMV may be restriction digested and subjected to Southern analysis, electrophoresis or the like, to detect the presence of the ORF insert in the virus.

The modified virus may then be serially passaged onto fresh host cells, e.g. human diploid lung or foreskin fibroblasts to provide for virus stocks.

The subject ORF containing CMVs may be used in humans or in tissue culture cells. They may be used for infecting cells in culture for production of a variety of products such as antigens from a variety of pathogens, or other products as indicated above. For use as a vaccine, the vaccine may be stored at -70°C in a solution of 70% sorbitol and distilled water (one volume of virus obtained at maximum of virus titer from cell cultures to one volume of sorbitol solution). The titer may be obtained by plaque assay or by employing antibodies to CMV, which are readily available (see U.S. Patent No. 3,959,466). Vaccines can be administered intradermally by using from about $1 \times 10^3$ to $5 \times 10^5$ plaque forming units in 0.1 to 0.2 ml.

One or more vaccinations may be given, usually at intervals of at least about two weeks. The blood from the vaccinated host may be isolated and screened for the presence of neutralizing antibodies to CMV and the antigen of interest, which was expressed by the one or more ORFs introduced into the CMV.

The ORF containing CMV may be used for the production of antibodies by employing the virus infected cells as immunogens and injecting them into appropriate hosts, e.g. mice, where neutralizing antibodies may then be obtained specific for the polypeptide expressed by the ORF(s). The ORF may be inserted into mouse CMV, rat CMV, guinea pig CMV, Simian CMV, bovine CMV, equine CMV or any other CMV and used to infect the homologous host to raise antibodies. While the human host is the primary host of interest, the subject invention may be used with any attenuated CMV strain as a vaccine for the host susceptible to such CMV.

EXPERIMENTAL

The following examples are offered by way of illustration and not by way of limitation.

In Fig. 1 is depicted the sequence arrangement of the CMV (Towne) genome showing L and S components in the prototypic orientation. Each component is composed of a unique sequence (thin lines) bracketed by inverted repeats of ab-b'c'-ca

(thick lines). The a is a terminal direct repeat with an inverted copy (a') at the LS junction.

The expanded region shows the HindIII K and O fragments and sequences used to construct pON256. pON256 was made by inserting a 5.9 kbp EcoRI partial fragment from pON241 (Spaete and Mocarski, J. Virol. (1985) 56:135) into the unique EcoRI site at the 5' edge of the 3' flanking region of the major β-gene carried by pON227. The EcoRI partial fragment contained the β gene promoter/regulatory sequences fused to lacZ/SV40 sequences. pON227 was derived as a BamHI to KpnI fragment of pON301 (Spaete and Mocarski, J. Virol. (1985) 54:817). In the process of digestion by T4 Pol (P-L Biochemical) and ligation to a filled in BamHI site, the KpnI site was converted to a BamHI site. pON227 contains 1.6 kbp of CMV β-gene sequences as depicted in Fig. 1. This 1.6 kbp of β-gene sequence provided the 3' flanking sequence for the lacZ expression construct.

DNA probes were employed in Southern blot analyses with the resulting construct as depicted in Fig. 1 RC256. The lacZ specific probe carried in pON1 is indicated by an arrow and the diagonally striped block. The a sequence probe, pON208, is indicated by a white box at the end of the HindIII K fragment. Direction of the 2.7 kb CMV β transcript is shown by a dotted arrow. Direction of the 5.0 kb lacZ transcript is depicted by an arrow and the deletion adjacent to the insert is depicted by a Δ and broken line.

RC256 was isolated after mixing 2.5 μg of pON256 (linearized with BstEII) and 20 μg of CMV (Towne) DNA and cotransfecting the mixture onto human fibroblast (HF) cells using calcium phosphate precipitation and glycerol shock (Spaete and Mocarski, J. Virol. (1985) 54:817). The progeny of a cotransfection were serially diluted and passaged onto fresh cells and the subsequent plaques were overlaid with 150μg/ml X-gal (Sigma), 0.5 agarose, 10% Nu serum (Collaborative Research) in Dulbecco's modified minimal essential medium (Gibco). Perspective recombinants were identified by the appearance of blue plaques 6 to 24 hours after overlay. Blue plaques were picked, sonicated, serially diluted and passaged onto fresh cells a total of three times. High tiltered virus stocks and viral DNA were prepared for analysis as described in Spaete and Mocarski, (1985), supra.

RC256 DNA was prepared from extracellular virions by digesting with proteinase K (100 μg/ml) (E. Merck), in the presence of 0.1% SDS and subsequent gentle extraction with phenol and ether. Restriction digests were performed according to the manufacturers specifications (N. E. Biolabs), the digests were separated by 0.5% agarose gel electrophoresis, transferred to nitrocellulose and probed with $^{32}$P-labeled DNA. A number of different digests were used. The changes in terminal and junction fragments as compared to original terminal and junction fragments supported the presence of the insert. A deletion was observed of sequences 5' to the insert which is indicated in Fig. 1 as a Δ.

DNA was prepared and the genome structure was analyzed by Southern blot hybridization (Southern, J. Mol. Biol. (1975) 98:503). HindIII and ClaI digests of the recombinant RC256 DNA were probed with lacZ specific sequences (pON1 [Spaete and Mocarski, J. Virol. (1985) 56:135]) as well as with CMV specific sequences flanking the insertion or deletion at the insertion site.

The analyses showed that lacZ was inserted into the CMV genome within the β gene, as expected, but only one copy of this gene was disrupted and a deletion of approximately 6 kbp had occurred close to the insertion site (Fig. 1). Because of the inversion of the L and S components of the RC256 genome, three new HindIII fragments were identified by hybridization to the lacZ probe, one L terminal (T*) and two L-S junction (J*) fragments. One copy of the CMV (Towne) HindIII K fragment was disrupted by the insertion while one remained unaffected.

ClaI digestion of CMV (Towne) DNA resulted in four separate terminal fragments (7.6 kbp, $T_1$; 13 kbp, $T_2$; 15 kbp, $T_3$ and 26 kbp, $T_4$) and four separate junction fragments (20.6 kbp, $J_1$, 22.6 kbp, $J_2$, 39 kbp, $J_3$; 41 kbp, $J_4$). Because ClaI cut outside the inverted repeats of the L and S components this enzyme was used to establish the structure of RC256.

As with HindIII digestion one T* and two J* ClaI fragments were identified by hybridization using the pON1 probe. ClaI digests of CMV (Towne) and RC256 were hybridized with pON208, which is an a sequence specific probe that hybridizes to all L-S junction and terminal fragments. This probe hybridized to the four junction ($J_1$-$J_4$) and four terminal ($T_1$-$T_4$) ClaI fragments in CMV (Towne) DNA digests. However, in RC256 digests $J_4$, $J_2$, and $T_3$ were missing and replaced with the new junction and terminal fragments. This was confirmed using pON227 as a probe, which is specific for 3' flanking sequences which hybridized to $T_2$, T*, $J_1$, $J_3$, and the two J* ClaI fragments.

A probe derived from a 315 bp MluI/EcoRI fragment representing sequences internal to the β gene deleted during the construction of pON256 was used to confirm the loss of $T_3$, $J_2$, and $J_4$ fragments from the RC256 genome.

DNA restriction patterns of ethidium bromide stained gels revealed the absence of HindIII 0 fragment due to the loss of DNA spanning the HindIII K/O site and the presence of a new HindIII fragment of 6.7 kbp (indicated by a Δ in Fig. 1.) Furthermore, XbaI O, BamHI G, and EcoRI O fragments, expected to be present in genome digests were absent.

ClaI restriction digests were hybridized with pON258 (Fig. 1) as a probe 5' flanking sequence (pON258 was constructed by insertion of a 180 bp HindIII to AluI fragment representing the 5' leader from pON241 into the HindIII/HincII polylinker of pGEM1 (Promega)). A 4.8 kbp fragment was detected, which represents a new fusion fragment resulting from the deletion of 6 kbp of sequences upstream of a β gene (Fig. 1).

The size and position of the deletion were established by Southern blot analyses of EcoRI, XbaI, HindIII, BamHI, BglII, BstEII, DdeI, KpnI, PvuII, SalI and XhoI digests, alone or in combinations using pXbaO (Thomsen and Stinski, Gene (1981) 16:207) and pON258 probes. These analyses indicated that the deletion had occurred farther than 1755 bp upstream of the transcription start site, suggesting that sufficient 5′ proximal sequences to drive the regulated expression of βgal were intact. These analyses also revealed that a proportion (5%) of the viral genomes in this stock have "repaired" these deleted sequences, suggesting a process of gene conversion previously observed in other herpesvirus repeats.

To demonstrate the transcription of lacZ specific sequences in RC256-infected cells, the following study was carried out.

The transcriptional activity of RC256 was examined using northern blot analysis. .5 µg RNA was prepared at 24h post infection (hpi) from cells infected with RC256 and probed to reveal the presence of a 5.0 kb transcript with homology to lacZ sequences (Geballe et al., J. Virol. (1986) 46:865). This transcript was identical in size to the transcript observed after transient introduction of a related construct (pON241) into HF cells. The size of the transcript suggested that transcription continued beyond the SV40 polyA addition signal present at the 3′ flank of lacZ and into the "late" SV40 sequences where appropriate processing signals could be used. The second uninterrupted copy of the β gene expressed a wild-type 2.7 kb transcript that was detected using the 315 bp MluI/EcoRI probe described above.

In order to examine levels of gene expression from the recombinant, cells infected with the wild-type or recombinant strains were radiolabeled for two hours with 200 µCi/ml [35S]methionine at 72 hpi. Proteins were prepared by cell solubilization in the presence of β-mercaptoethanol and SDS, and separated by SDS/polyacrylamide (7.5% gel) electrophoresis (SDS/PAGE), transferred to nitrocellulose filters and autoradiographed (Hutchinson et al., Virology (1986) 155:160; McDonough et al., J. virol. (1985) 53:711, Wathen and Stinski, J. Virol. (1982) 41:462). β-gal was expressed as a prominent gene product in RC256 infected cells.

The species designated as the βgal monomer is based on its relative mobility as well as by Western blot analysis using high-titer rabbit anti-βgal antibody (Cooper Biochemical). At late times in infection, the levels of expression of βgal from this recombinant approached 0.2-1% of total infected cell protein. This value was derived by measuring the moles of substrate (ONPG) cleaved per minute under conditions defined by Rotman in The Lactose Operon, J. Beckwith and D. Zipser, ed., (CSH NY 1972 pp. 279-289), where one unit of β-galactosidase corresponds to 2.6 x 10⁹ molecules of tetrameric enzyme.

The kinetics of βgal expression from RC256 were studied by radiolabeling cells with [35S]methionine for 2h at 2, 6, 10, 24, and 48 hpi and separating proteins by SDS/PAGE. Both autoradiographic as well as western blot data indicated that βgal synthesis began by 6 and 8 hpi and reached maximal rates by 10 to 12 hpi. Furthermore, βgal expression was blocked by cycloheximide treatment. These observations are consistent with previously published transient assays in the designation of this gene's kinetic class.

It is evident from the above results, that CMV can be used for the introduction of exogenous proteins into a human host cell for expression of the exogenous protein in active form, so as to substantially mimic the natural protein as prepared by its native host. In this way, vaccines are provided which can be used to vaccinate individuals for protection not only against CMV, but a wide variety of other pathogens without concern as to infection by such pathogens. Thus, a safe and effective means is provided for producing antigens to induce immunogenic response. In addition, the viruses may be used for producing a wide variety of products in cell culture or in vivo, where cells lacking the particular phenotype may be provided with the phenotype by infection with the CMV. The CMV may also be used to modulate activity of a particular gene by providing for an antisense transcript, which would prevent the translation of sense transcripts made by the host. Thus, the CMV provides for a broad spectrum of activities by introducing a wide variety of genetic capabilities into the intact mammalian, particularly human, host or mammalian cell culture.

Although the foregoing invention has been described in some detail by way of illustration and example for purposes of clarity of understanding, it will be obvious that certain changes and modifications may be practiced.

## Claims

1. An attenuated cytomegalovirus capable of cellular infection comprising a foreign gene capable of expression in a CMV infected host cell.

2. An attenuated cytomegalovirus according to Claim 1, wherein said foreign gene encodes a pathogenic protein or fragment thereof.

3. An attenuated cytomegalovirus according to Claim 2, wherein the pathogenic protein or fragment thereof is an envelope glycoprotein or fragment thereof.

4. An attenuated cytomegalovirus according to Claim 2, wherein the pathogenic protein or fragment thereof is a surface membrane protein of a cellular microorganism or fragment thereof.

5. An attenuated cytomegalovirus capable of cellular infection comprising an expression cassette inserted into the b sequence and at least one functional β-gene, said expression cassette comprising transcriptional and translational regulatory initiation and termination regions and a foreign gene under the regulation of said regions, said regulatory regions functional in a cytomegalovirus infected human host cell.

6. An attenuated cytomegalovirus comprising a foreign protein in at least one of the cytomegalovirus capsid and envelope.

7. An attenuated cytomegalovirus according to Claim 6, wherein said foreign protein is an envelope protein or fragment thereof.

8. An attenuated cytomegalovirus according to Claim 6, wherein said foreign protein is a surface membrane protein or fragment thereof of a pathogen.

9. A vaccine comprising an effective amount of an attenuated cytomegalovirus according to Claim 6, Claim 7 or Claim 8 and a physiologically acceptable carrier.

10. A DNA construct comprising in the direction of transcription, a sequence from the b sequence of CMV, a transcriptional and translational regulatory initiation region, an open reading frame from other than cytomegalovirus under the transcriptional control of said initiation region, a transcriptional and translational regulatory termination region, a sequence from the b sequence of CMV.

11. A DNA construct according to Claim 10, wherein said ORF encodes a viral protein.

12. A DNA construct according to Claim 11 wherein said viral protein is a capsid or envelope protein.

Claims for the following contracting states: ES, GR

1. An attenuated cytomegalovirus capable of cellular infection comprising a foreign gene capable of expression in a CMV infected host cell.

2. An attenuated cytomegalovirus according to Claim 1, wherein said foreign gene encodes a pathogenic protein or fragment thereof.

3. An attenuated cytomegalovirus according to Claim 2, wherein the pathogenic protein or fragment thereof is an envelope glycoprotein or fragment thereof.

4. An attenuated cytomegalovirus according to Claim 2, wherein the pathogenic protein or fragment thereof is a surface membrane protein of a cellular microorganism or fragment thereof.

5. An attenuated cytomegalovirus capable of cellular infection comprising an expression cassette inserted into the b sequence and at least one functional β-gene, said expression cassette comprising transcriptional and translational regulatory initiation and termination regions and a foreign gene under the regulation of said regions, said regulatory regions functional in a cytomegalovirus infected human host cell.

6. An attenuated cytomegalovirus comprising a foreign protein in at least one of the cytomegalivirus capsid and envelope.

7. An attenuated cytomegalovirus according to Claim 6, wherein said foreign protein is an envelope protein or fragment thereof.

8. An attenuated cytomegalovirus according to Claim 6, wherein said foreign protein is a surface membrane protein or fragment thereof of a pathogen.

9. A method of making a vaccine comprising an effective amount of an attenuated cytomegalovirus according to Claim 6, Claim 7 or Claim 8 and a physiologically acceptable carrier.

10. A method of making a DNA construct comprising in the direction of transcription, a sequence from the b sequence of CMV, a transcriptional and translational regulatory initiation region, an open reading frame from other than cytomegalivirus under the transcriptional control of said initiation region, a transcriptional and translational regulatory termination region, a sequence from the b sequence of CMV.

11. A DNA construct according to Claim 10, wherein said ORF encodes a viral protein.

12. A DNA construct according to Claim 11 wherein said viral protein is a capsid or envelope protein.

0277773

17·03·88

FIG. IA

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 176 170 (INSTITUT MERIEUX)<br>* Whole document *<br>--- | 1-12 | C 12 N 15/00<br>A 61 K 39/245 |
| D,Y | US-A-4 058 598 (STERN)<br>* Whole document *<br>--- | 1-12 | |
| D,Y | CELL, vol. 46, 12th September 1986, pages 865-872, Cell Press; A.P. GEBALLE et al.: "A cis-acting element with the 5' leader of a cytomegalovirus beta transcript determines kinetic class"<br>* Whole document *<br>--- | 5,10 | |
| D,A | FR-A-2 267 117 (THE WISTAR INSTITUTE)<br>--- | | |
| A | VACCINE, vol. 3, March 1985, pages 45-48, Butterworth & Co. (Publishers) Ltd; D. CAVANAGH: "Viral and bacterial vectors of immunogenes"<br>--- | | |
| A | GENE, vol. 47, no. 2/3, 1986, pages 193-199, Elsevier Science Publishers B.V., Amsterdam, NL; D. PANICALI et al.: "Vaccinia virus vectors utilizing the beta-galactosidase assay for rapid selection of recombinant viruses and measurement of gene expression"<br>--- | | TECHNICAL FIELDS SEARCHED (Int. Cl.4)<br><br>C 12 N<br>A 61 K |
| P,X | PROC. NATL. ACAD. SCI. USA, vol. 84, October 1987, pages 7213-7217; R.R. SPAETE et al.: "Insertion and deletion mutagenesis of the human cytomegalovirus genome"<br>----- | 1-12 | |

The present search report has been drawn up for all claims

| Place of search | Date of completion of the search | Examiner |
|---|---|---|
| THE HAGUE | 31-03-1988 | SKELLY J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
 
& : member of the same patent family, corresponding document

EPO FORM 1503 03.82 (P0401)